# EUROPEAN PATENT SPECIFICATION

(11) **EP 0 575 357 B1**
(45) Date of publication and mention of the grant of the patent: **18.09.2002**
(21) Application number: 92904833.8
(22) Date of filing: 02.01.1992
(51) Int. Cl.: C12N 15/12, C07K 14/00, A61K 38/00, C07K 2/00, A61K 38/04

(54) **HUMAN FOLLICLE STIMULATING HORMONE RECEPTOR**
Menschlicher Rezeptor für das Follikel-stimulierendes Hormon.
RECEPTEUR DE L'HORMONE FOLLICULOSTIMULANTE HUMAINE

(30) Priority: 15.03.1991 US 670085
(43) Date of publication of application: 29.12.1993
(60) Divisional application: 01202809.8
(73) Proprietor: Applied Research Systems ARS Holding N.V., Curacao (AN)
(72) Inventor: KELTON, Christie, Ann, Hopkinton, MA 01748 (US); CHENG, Shirley, Vui, Yen, Wellesley, Massachusetts 02181 (US); NUGENT, Noreen, Patrice, Framingham, MA 01701 (US); SCHWEICKHARDT, Rene, Lynn, Medfield, Massachussetts 02052 (US)
(74) Representative: Ellowicz, Leo, Drs.
(86) International application number: PCT/US92/00122
(87) International publication number: WO 92/016620

(56) References cited:
- WO-A-90/13643
- US-A- 4 921 808
- MOLECULAR ENDOCRINOLOGY vol. 4, no. 4, 1990, pages 525 - 530; Sprengel, Rolf; Braun, Thomas; Nikoli
- BIOCHEMICAL AND BIOPHYSICAL RESEARCH COMMUNICATIONS. vol. 175, no. 3, 29 Mar

## Description

### Background of The Invention

This invention relates to human follicle stimulating hormone receptor and its synthesis by recombinant DNA techniques.

Follicle stimulating hormone (FSH) is a pituitary-derived heterodimeric glycoprotein hormone which shares structural similarities with luteinizing hormone (LH) and thyroid stimulating hormone (TSH), both of which are also produced in the pituitary gland, and chorionic gonadotropin (CG), which is produced in the placenta. The hormones are relatively large (28-38 kilodaltons) and are composed of a common α subunit non-covalently bound to a distinct β subunit that confers receptor binding specificity.

The cellular receptors for these hormones are known to be members of the G protein-coupled class of membrane-bound receptors which, when activated, stimulate an increase in the activity of adenylyl cyclase. This results in an increase in the level of the intracellular second messenger adenosine 3',5'-monophosphate (cAMP), which in turn causes increased steroid synthesis and secretion. Hydrooathicity plots of the amino acid sequences of these receptors reveal three general domains: (1) a hydrophilic amino-terminal region, considered to be the amino-terminal extracellular domain, (2) seven hydrophobic segments of membrane-spanning length, considered to be the transmembrane domain, and (3) a carboxy-terminal region which contains potential phosphorylation sites (serine, threonine, and tyrosine residues), considered to be the carboxy-terminal intracellular or cytoplasmic domain. The glycoprotein hormone receptor family is distinguished from other G protein-coupled receptors, such as the β2-adrenergic, rhodopsin, and substance K receptors, by the large size of the hydrophilic amino-terminal domain, which is involved in hormone binding.

The FSH receptor is expressed on testicular Sertoli cells and ovarian granulosa cells. While there has been a recognized need for providing essentially pure human FSH receptor, purification of naturally derived preparations is not practical and would not likely be sufficient to permit determination of the amino acid sequence. Recently, one group has cloned the cDNA encoding the rat FSH receptor, deduced the amino acid sequence, and expressed it in mammalian cells (WO 90/13643 and Sprengel, Mol. Endocrinol. 4:525, 1990). Another group, attempting to clone the TSH receptor, apparently also cloned and identified a portion of the transmembrane region of the human FSH receptor (Parmentier, Science 246:1620, 1989).

### Summary of The Invention

The present invention embraces essentially pure human FSH receptor, or a fragment or mutant thereof which binds FSH, according to present claims DNA encoding said receptor, fragment or mutant, expression vectors comprising said DNA, cell transfected with said expression vector, and methods of producing said receptor, fragment or mutant by culturing said transfected cells. The present invention also includes pharmaceutical compositions comprising said receptor, fragment or mutant, as well as methods of treating patients with such compositions to reduce endogenous FSH bioactivity. An improved assay for human FSH using the receptor or FSM- binding fragments thereof is also disclosed.

### Brief Description of The Drawings

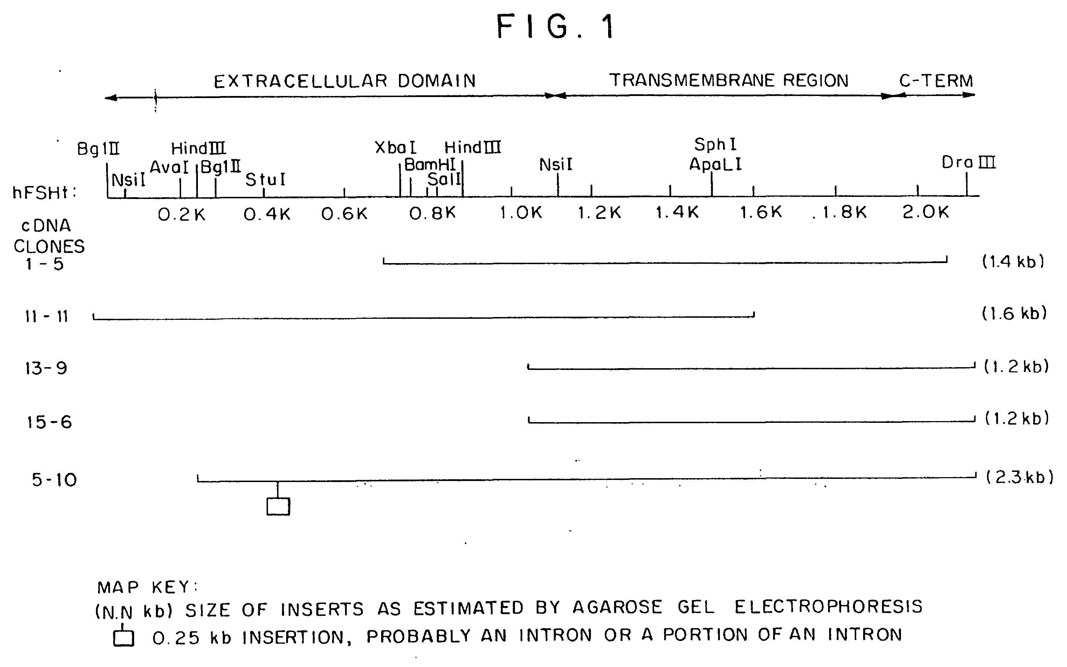

Figure 1 is a map of the human FSH receptor cDNA clones. A partial restriction endonuclease map is shown which was determined by combining DNA sequence data derived from portions of each of the five clones. Size increments of 0.2 kilobase pair (kb) are marked. The regions to which the clones correspond are indicated by the solid lines below the restriction endonuclease map. The clone designation and the approximate size in kb are indicated to the left and right of each solid line, respectively. Approximate locations of the amino-terminal extracellular domain, transmembrane domain, and carboxy-terminal intracellular domain of the encoded protein are indicated by the dashed arrows above the restriction endonuclease map. The position of an insertion (probably an intron or a portion of an intron) in the 5-10 clone is indicated by the open box.

Figures 2A and 2B depict the strategy used for engineering a "full-length" human FSH receptor cDNA for the purpose of expression of the encoded protein in a mammalian cell line.

Figure 3 is a diagram of the expression vector plasmid used to develop CHO-DUKX cell lines stably transfected with the human FSH receptor.

Figure 4 is a diagram of the expression vector plasmid used to develop Y1 cell lines stably transfected with the human FSH receptor.

Figure 5 illustrates intracellular cAMP levels measured in the CHO-DUKX cell line CHOHFSHR4Q-13, after treatment with varying doses of either FSH (circles), or LH (triangles). Each point represents the mean value. Standard errors are shown by the vertical bars.

Figure 6 illustrates progesterone levels measured in the culture medium of the Y1 cell line Y1HFSHR4-38, after treatment with varying doses of FSH. Vertical bars depict the standard error in the progesterone measurements at each FSH dose.

### Detailed Description of the Invention

As used herein, human FSH receptor, and its FSH-binding fragments or mutants, refers to polypeptides which are capable of recognizing and selectively binding with human FSH and which produce no significant immunological response in humans. Thus, the present invention includes human FSH receptor having the amino acid sequence depicted in Sequence ID No:2, as well as FSH-binding fragments or mutants thereof which retain a very high degree of homology (i.e. at least 95% identity) with the depicted amino acid sequence, or with at least the extracellular portion thereof. Fragments of the human FSH receptor embraced by the present invention include those fragments remaining after the cytoplasmic and/or the transmembrane domains have been deleted from the full polypeptide. An especially preferred fragment embraced by the present invention is the amino-terminal extracellular portion comprising approximately amino acids 1 to 349 of the human FSH receptor amino acid sequence shown in Sequence ID No:2. Since some of the loops which span the transmembrane regions (listed in features section, Sequence ID No:2) are also extracellular, these may be linked (e.g. through appropriate spacer molecules) to a fragment containing the amino-terminal extracellular portion to improve binding. The polypeptide may be glycosylated, as in the natural receptor, or may be partially or completely deglycosylated.

It is further contemplated that only a portion of the above-described amino-terminal extracellular domain may be effectively utilized to bind FSH since it is very likely that the complete extracellular domain is not necessary for this purpose. Thus, a fragment which is somewhat shorter than the 349 amino acids of the complete extracellular domain may be readily produced and tested for effective binding to FSH. So long as the FSH-binding region of the extracellular portion is maintained intact, the length of the overall fragment utilized is not critical. For this reason, it is also expected that non-interfering amino acids can be added to either end of the extracellular domain, or FSH-binding fragment thereof, without adversely affecting the FSH-binding capacity of the polypeptide. Accordingly, this invention embraces a fragment of the human FSH receptor which comprises a substantial portion of the extracellular domain and which retains substantially the same FSH-binding characteristics as the complete extracellular domain.

Mutant forms of the above-described receptor and fragments are also within the scope of the present invention. Such mutants include conservative substitutions in one to ten amino acid residues, the location and nature of such substitutions being selected so as not to significantly degrade the FSH-binding characteristics of the receptor or fragment thereof which is unmodified.

A pure human FSH receptor, or a fragment or mutant thereof, is prepared by isolating and cloning the DNA encoding it from a cDNA or genomic library, ligating the DNA into a vector, transfecting host cells with the vector, culturing transfected host cells under conditions which permit expression of the receptor, fragment or mutant, and recovering the receptor, fragment or mutant from the culture.

The DNA which is used to make expression vectors may be genomic DNA or cDNA encoding human FSH receptor, and may contain regions which enhance expression, such as introns, promoters, enhancers, etc. The DNA may be readily modified by substitution, deletion or insertion of nucleotides (e.g. by site-specific mutagenesis) that do not adversely affect the biological or FSH-binding activity of the expressed protein. For example, conservative substitutions (mutations) which alter from one to ten amino acids may be made without adversely affecting the overall structure and activity of the expressed protein (mutein). Likewise, certain portions of the DNA, such as those portions which code for the cytoplasmic and/or transmembrane domains, may be deleted so that only a fragment, such as the soluble extracellular domain, of the protein is expressed. The human FSH receptor or FSH-binding fragment or mutant thereof may also be expressed as a fusion protein. One such fusion protein would include a polypeptide at the carboxy-terminus which would confer characteristics which would facilitate purification of the protein, or immobilization of the purified protein on a solid substrate for use in FSH assays or FSH purification protocols. Another such fusion protein would include a cleavable polypeptide at the amino-terminus which would facilitate expression.

Human FSH receptor produced in accordance with the present invention is essentially pure, meaning that it is substantially free of biological adventitious agents normally associated with FSH receptor extracted from natural sources, such as, for example, bacteria, viruses, and other proteins. The receptor may be formulated into pharmaceutical compositions by mixing with suitable pharmaceutically acceptable carriers in a manner known to those skilled in the art.

Generally, pharmaceutical compositions may be formulated for oral, parenteral (including subcutaneous, intramuscular, and intravenous), vaginal, rectal, buccal (including sublingual), transdermal or intranasal administration. Compositions for parenteral administration are normally in the form of a liquid solution, dispersion, or emulsion, preferably isotonic; for vaginal or rectal administration, as a cream or suppository; for oral or buccal administration, as a tablet or capsule; and for intranasal administration, as a powder, nasal drop or aerosol. Various slow release, depot implant or injectable dosage forms may be utilized. The active component may also be incorporated into polymer matrices, liposomes and microspheres to control delivery.

These compositions may conveniently be administered in unit dosage form and may be prepared by any of the methods well-known in the pharmaceutical art. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. Formulations for vaginal or rectal administration, e.g. suppositories, may contain as excipients, for example, polyalkyleneglycols, vaseline, cocoa butter, and the like. Formulations for nasal administration may be in powder form and contain as excipients, for example, lactose or dextran, or may be aqueous or oily solutions for administration in the form of nasal drops or metered spray. For buccal administration, typical excipients include sugars, calcium stearate, pregelatinated starch and the like. One or more surfactant acids or salts can be added to the solution or powder formulation. Suitable pharmaceutically acceptable surfactant salts will be those which retain the phenomenon of enhanced peptide absorption, as well as the compound's surfactant characteristics and which are not deleterious to the subject or otherwise contraindicated.

The dosage of active ingredient administered, as well as the route and frequency of administration, will obviously depend upon the needs and condition of the patient being treated, the therapeutic effect which is sought, and the judgement of the doctor conducting the treatment.

Pharmaceutical compositions comprising human FSH receptor, or FSH-binding fragments or mutants thereof, may be administered to a patient in therapeutically effective doses to bind with endogenous circulating FSH in the patient and thereby control the available level of bioactive FSH. Thus, pharmaceutical compositions of the present invention are effectively utilized to reduce endogenous FSH bioactivity. In a female patient, such treatment may be effectively used to prevent follicle growth and maturation, thereby preventing pregnancy. In a male patient, such treatment may be effectively used to prevent spermatogenesis. A particularly suitable pharmaceutical composition for the above purpose comprises a fragment of the human FSH receptor which comprises the amino-terminal extracellular domain or a substantial portion thereof with the same FSH-binding characteristics.

Essentially pure human FSH receptor may also be advantageously utilized in conventional receptor assays for FSH, such as, for example, that disclosed in Reichert, Endocrinology 94:483, 1974. Substitution of the pure receptor of the present invention, or a fragment thereof which binds FSH, will substantially improve the consistency and performance of such assays. A fragment comprising the amino-terminal extracellular domain or an FSH-binding fragment and/or fusion protein thereof can also be linked to an affinity column to purify FSH from fluids, extracts, etc.

The receptor can be incorporated into a stable cell line, preferably a eukaryotic cell line, and most preferably a mammalian cell line, which is capable of producing a measurable biological response upon stimulation of the receptor. Measurement of cell response in the presence of FSH under assay (e.g. in test serum, plasma, culture media, tissue homogenates, etc.) would provide an indication of bioactivity, and thereby provide a highly useful diagnostic assay. Such a cell line could also be used to screen chemical libraries for substances that may interact with the FSH receptor or to test peptides or small proteins for their ability to bind to the FSH receptor in a rapid through-put screening system. An example of a rapid through-put screening system is one in which the binding of a ligand to the recombinant FSH receptor results in the production or blockage of production of a measurable product such as cAMP or progesterone. Such a system could be further enhanced by operatively linking an easily measured marker element such as one which produces bioluminescence (e.g. the luciferase gene) to the receptor signal transduction pathway (e.g. by using the cAMP response element).

A pure human FSH receptor or FSH-binding fragments or mutants thereof can also be used in x-ray crystallographic analysis to develop molecular models. Such models are useful in defining the tertiary structure of the hormone-binding domains of the human FSH receptor. Such information would provide insight into the structure of the actual regions of contact between FSH and its receptor, thus aiding the design of peptides which have FSH agonistic or antagonistic activity.

The recombinant techniques suitable for producing the proteins and DNA of the present invention, including identification of suitable mutation techniques, vectors, host cells, culture conditions, etc., are well-known to those skilled in the art and are adequately described, for example, in US 4,761,371 and WO 88/09818. The experimental protocols, bacterial and bacteriophage culture media and chemical solutions used in the examples below are described in detail in Sambrook, "Molecular Cloning: A Laboratory Manual," 2nd ed., Cold Spring Harbor Laboratory Press, 1989, unless otherwise referenced.

### Example 1. Isolation and Characterization of Human FSH Receptor cDNA Clones

### Library screening.

A rat FSH receptor cDNA clone similar to that described by Sprengel, Mol. Endocrinol. 4:525, 1990 was obtained from Dr. William Moyle, University of Medicine and Dentistry of New Jersey-Robert Wood Johnson Medical School. This cDNA clone was inserted into the SV40 late expression vector pSVL (Pharmacia LKB, product number 27-4509-01) and was designated pSVLFSHR. A 2.1 kb DNA fragment containing the region corresponding to the complete rat FSH receptor protein coding region was excised from the plasmid using the restriction endonuclease sites XbaI and BamHI. The digested DNA was size-fractionated by gel electrophoresis, and the 2.1 kb rat FSH receptor fragment was purified by electroelution from the gel. This purified DNA fragment was used as a probe for library screening to identify human FSH receptor cDNA clones.

A lambda gtll cDNA library, constructed from RNA extracted from human testis, was purchased from Clontech, Palo Alto, CA (catalogue number HL1010b) and was amplified prior to use. Twenty aliquots of the amplified library, equivalent to approximately 7.5 x 10⁴ plaque forming units (pfu), were each adsorbed to about 0.5 milliliters (ml) of a plating suspension of E. coli strain Y1088. The suspension had been prepared by growing an overnight culture of Y1088 at 37°C in NZYM or LB supplemented with 0.2% maltose and 10 millimolar (mM) MgSO₄, pelleting the cells, and then resuspending them in 10 mM MgSO₄ to an O.D.₆₀₀ of 0.5. About 6.5 ml of molten NZYM top agarose (0.7%), at a temperature of 48°C, was added to each phage/cell suspension and the resulting mixture was poured onto one of twenty 150 millimeter (mm) NZYM agar plates (prewarmed to 42°C). The total number of phage plated for the primary screen was about 1.5 X 10⁶. After a 4 hour incubation at 42°C, followed by chilling at 4°C for several hours, duplicate nitrocellulose filter (Millipore) plaque lifts were generated from each plate according to the procedures of Benton and Davies (Science, 196:180, 1977). The random oligonucleotide priming procedure of Feinberg and Vogelstein (Anal. Biochem. 137:266, 1983) was used to generate, from the template rat FSH receptor DNA fragment described in the preceding paragraph, a ³²P-labeled probe of a specific activity of 1-2 X 10⁹ counts per minute (cpm)/microgram (µg). Prehybridization of the nitrocellulose filter phage lifts was done in a buffer containing 50% formamide, 5X SSC [1X SSC is 0.15 molar (M) sodium chloride, 0.015 M sodium citrate], 20 mM sodium phosphate buffer, pH 7.2, 10X Denhardt's reagent (50X Denhardt's reagent is 1% Ficoll, 1% polyvinylpyrrolidone and 1% bovine serum albumin) and 100 µg/ml tRNA at 37°C for about 6 hours. Hybridization of the filters was done in the same buffer except that the rat FSH receptor DNA probe was added at a concentration of about 3 X 10⁶ cpm/ml buffer. After hybridization for 16-24 hours at 37°C, excess probe was washed from the filters in 2X SSC, 0.1% SDS at room temperature for 30 minutes, then 0.2X SSC, 0.1% SDS at 37°C for 60 minutes. The filters were subsequently exposed to XAR film (Kodak) overnight at -70°C. Six duplicate positives were identified from the primary library screen. With the wide end of a pasteur pipette, plaque-containing agar plugs were removed from the 150 mm plates in the regions in which the positive clones were located. The phage were eluted by soaking the plugs in SM. The suspended phage were then replated onto 150 mm NZYM agar plates as described for the primary screen. Plates which contained about 500 pfu per plate were selected for secondary screening. The procedures used for filter lifts and filter hybridizations for the secondary screen were the same as described for the primary screen.

Following the secondary screen, 5 putative human FSH receptor positives were identified and isolated as purified λgt11 bacteriophage clones. They were assigned the following designations: 1-5, 5-10, 11-11, 13-9, and 15-6.

### Determination of the DNA sequences of the putative human FSH receptor cDNA clones.

Bacteriophage DNA was prepared from each of the λgt11 cDNA isolates using the plate lysate method described in Sambrook, "Molecular Cloning: A Laboratory Manual," 2nd ed., Cold Spring Harbor Laboratory Press, 1989, page 2.118. The bacteriophage DNA was digested with EcoRI restriction endonuclease, and then size-fractionated in an agarose gel for purification of the cDNA insert fragments. The purified cDNA inserts were subcloned into the EcoRI site of pUC18 in order to facilitate subsequent cloning and sequencing manipulations. Double-stranded plasmid DNA was purified from 5 ml cultures of the host strain E. coli MC1061 by the small-scale alkaline lysis method (Sambrook, ibid. p. 1.25), then further purified by passage over Elutip-d columns (Schleicher & Schuell, Keene, NH) using the manufacturer's protocol. Half of the plasmid DNA obtained from each small-scale plasmid preparation was then denatured in 0.2 normal NaOH, 0.2 mM EDTA in a volume of 20 microliters (µl) at room temperature for 10 minutes. The denatured plasmid DNA was neutralized and ethanol precipitated by adding 7.5 µl of 7.5 M ammonium acetate and 110 µl 100% ethanol and chilling the mixture in liquid nitrogen. The DNA precipitate was pelleted by centrifugation in a microfuge for 10 minutes. The DNA pellets were washed in 70% ethanol and dried. Sequencing reactions were done with Sequenase T7 DNA polymerase (United States Biochemical) according to the manufacturer's specifications. Preliminary sequencing reactions were done with forward and reverse sequencing primers (Pharmacia LKB) for the pUC18 polylinker region. Data generated from preliminary sequencing was used to design human FSH receptor-specific sequencing primers. The primers were either synthesized on a model 391 Applied Biosystems DNA synthesizer or ordered from National Biosciences, Inc., Hamel, MN. Some DNA sequence data was obtained by subcloning smaller restriction endonuclease fragments of the original clones into pUC18 and repeating sequencing reactions with the forward and reverse primers.

Preliminary sequence data showed that none of the five cDNA isolates represented the full-length protein coding region of the human FSH receptor, but that the combined sequence data from the clones could be used to infer the complete protein sequence. A schematic diagram of the relative locations of each of the five clones in relation to a map of the complete human FSH receptor cDNA sequence is shown in figure 1. The complete human FSH receptor cDNA sequence, obtained by combining the sequence readings from the overlapping clones using the Genetics Computer Group (GCG) fragment assembly computer program, is depicted in Sequence ID No:1 and the inferred amino acid sequence is depicted in Sequence ID No:2. Analysis of the human FSH receptor DNA sequence resulted in the identification of a long open reading frame of 2085 nucleotides which encoded a protein of 695 amino acids. The human FSH receptor is therefore 3 amino acids longer than the rat FSH receptor. The overall percent identities between the rat and human FSH receptor DNA and protein sequences were determined using the GCG Bestfit program and were 87% and 90%, respectively. The extracellular amino-terminal hydrophilic portion of the human FSH receptor is estimated to be 349 amino acids in length, and shares 87% identity with the corresponding region of the rat FSH receptor. The seven membrane spanning regions of the two species, which are bridged by three extracellular and three intracellular loops, share 95% identity, and the carboxy-terminal intracellular regions share only 81% identity. The partial amino acid sequence published by Parmentier, Science 246:1620-1622, 1989, corresponds to amino acids 399 to 525 in Sequence ID No:2.

Clone 5-10 was a variant in that it contained an insertion of 0.25 kb after the T at nucleotide position 448 in Sequence ID No:1. The DNA sequence of the insertion was not similar to any portion of the rat or human FSH receptor DNA sequence and also was not similar to any known sequence in the Genbank or EMBL DNA sequence databases. The corresponding region in clone 11-11 did not contain this insertion. LH receptor cDNA clones isolated from a human thyroid cDNA library contain similar variations (Frazier, Mol. Endocrinol. 4:1264-1276, 1990). These variants are likely derived from incompletely and/or aberrantly spliced mRNA molecules. The presence of a 3' splice consensus sequence (CAG'G) at the 3' junction of the 5-10 insertion is evidence which supports this explanation.

The pUC18 plasmids harboring the 11-11 (called pHFSHR11-11), 15-6 (called pHFSHR15-6), and 5-10 (called pHFSHR5-10) cDNA inserts were deposited with the American Type Culture Collection (ATCC), Rockville, MD, on March 1, 1991 and have received the accession numbers ATCC68538, ATCC68540, and ATCC68539, respectively. These deposits were made in accordance with all of the requirements of the Budapest Treaty.

### Example 2. Construction of vectors for the expression of the full-length human FSH receptor in mammalian cells.

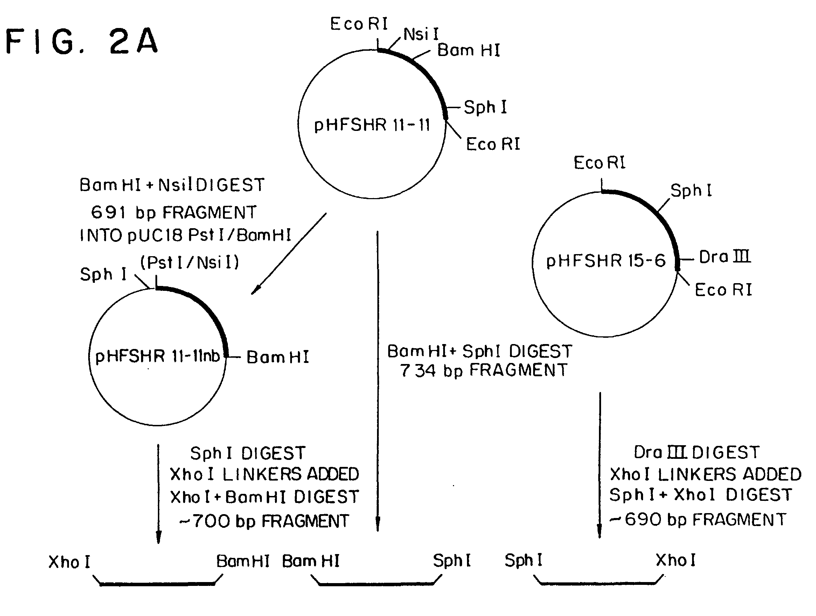

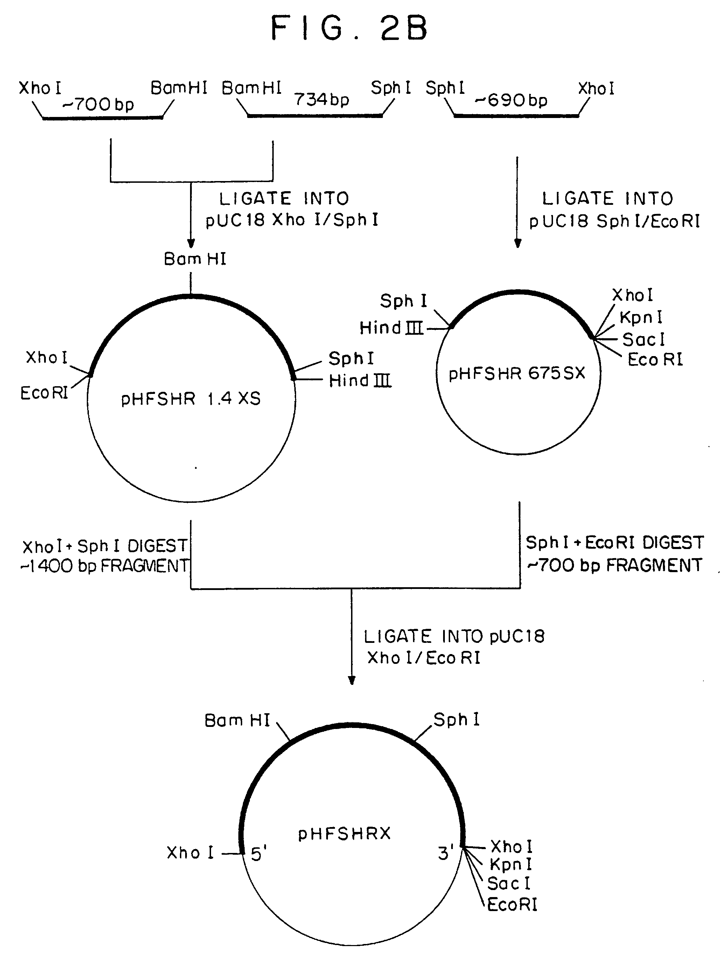

The strategy for engineering a human FSH receptor DNA construct for expression in mammalian cells is shown in Figures 2A and 2B. Refering to Figure 2A, the 5' 691 base pair (bp) NsiI-BamHI fragment which contained the start ATG was purified from pHFSHR11-11 and subcloned into pUC18 digested with PstI and BamHI. The resulting plasmid, pHFSHR11-11nb, was linearized with SphI. The termini were blunted by treatment with the Klenow fragment of DNA polymerase I (New England Biolabs, Beverly, MA). After ligation of XhoI linkers (New England Biolabs, Beverly, MA) to the blunted termini, the mixture was digested with XhoI and BamHI and the approximately 700 bp human FSH receptor 5' fragment was gel purified. A fragment from the middle region of the human FSH receptor cDNA was isolated from pHFSHR11-11 by digestion with BamHI and SphI and gel purification of the appropriate 734 bp piece. The 3' human FSH receptor fragment, which contained the TAA stop codon, was isolated from pHFSHR15-6 by first digesting the plasmid with DraIII, then blunting the termini in a reaction with T4 DNA polymerase. XhoI linkers were ligated to the blunted ends and the resulting mixture was digested with SphI and XhoI and the 3' 690 bp (approximately) SphI-XhoI fragment was isolated and purified.

Refering to Figure 2B, the 5' 700 bp XhoI-BamHI and middle 734 bp BamHI-SphI human FSH receptor cDNA fragments were subcloned into pUC18-XhoI (created in this laboratory by converting the SmaI site in the pUC18 polylinker to an XhoI site with XhoI linkers) digested with XhoI and SphI. The resulting plasmid, pHFSHR1.4xs, was digested with XhoI and SphI. The digested DNA was size-fractionated by electrophoresis in a 5-6% polyacrylamide gel and the approximately 1400 bp fragment containing the 5' and middle regions of the human FSH receptor cDNA was excised and purified by electroelution. The 3' SphI-XhoI fragment, which contains the TAA stop codon, was subcloned into pUC18-XhoI digested with SphI and XhoI. The resulting plasmid, pHFSHR675sx, was digested with SphI and EcoRI. The digested DNA was fractionated in a 5-6% polyacrylamide gel and the approximately 700 bp piece was excised and purified by electroelution. The complete human FSH receptor coding region was assembled by combining the 5' 1400 bp XhoI-SphI fragment with the 3' 700 bp SphI-EcoRI fragment in a ligation with pUC18-XhoI digested with XhoI and EcoRI. Correct assembly of the construction, called pHFSHRX, was verified by restriction endonuclease digestion and DNA sequencing. The completely engineered human FSH receptor 2.1 kb XhoI fragment, which contains approximately 18 bp of 5' noncoding sequence and 12 bp of 3' noncoding sequence in addition to the entire human FSH receptor protein coding region, was excised from pHFSHRX by XhoI digestion. The digested DNA was size-fractionated by electrophoresis in a 0.7% agarose gel. The 2.1 kb XhoI fragment was excised from the gel, purified by electroelution in a Little Blue Tank™ (ISCO) using the procedure recommended by the manufacturer, and inserted into the XhoI cloning sites of the mammalian cell expression vector plasmids. Plasmids containing the insert in the appropriate orientation for human FSH receptor mRNA transcription were selected by restriction endonuclease fragment analysis. Prior to introduction into mammalian cells, expression vector plasmid DNA was purified either by two sequential high-speed centrifugations through cesium chloride density gradients, or by using the plasmid maxi kit (Qiagen, Chatsworth, CA) according to the manufacturer's protocol.

Any suitable mammalian cell expression vector can be used for the expression of the human FSH receptor. The necessary vector plasmid components include the following: a eukaryotic promoter such as the mouse metallothionein I (MMT-I) promoter, Rous Sarcoma Virus promoter, or simian virus 40 early or late promoters for driving transcription of the human FSH receptor mRNA; a marker gene such as the neomycin resistance gene (Neo), dihydrofolate reductase (DHFR) gene, or multidrug resistance gene (MDR) for the selection of transfected cells; a polyadenylation signal (poly A), such as the SV40 early polyadenylation region for the 3' processing of the receptor RNA transcript; and a bacterial origin of replication and antibiotic resistance gene such as the pBR322 ori and the ampicillin resistance gene, to enable growth and propagation of the vector plasmid in a suitable E. coli strain. For some cell lines, it may be important to include an intron in the region which is transcribed into human FSH receptor encoding mRNA. It is preferable to place the intron between the promoter and the human FSH receptor cDNA insert, thereby positioning it in the 5' untranslated region of the human FSH receptor RNA transcript. Any suitable intron can be used. The intron used in our experiments was a 2 kb XbaI-PstI portion of intron A in the human glycoprotein α subunit gene (Fiddes, J. Mol. Appl. Genet. 1:13, 1981). In the expression vectors, it was inserted between the the MMT-1 promoter region and the human FSH receptor cDNA fragment. The truncated intron retained the endogenous splice acceptor, but the splice donor was supplied by a synthetic oligonucleotide.

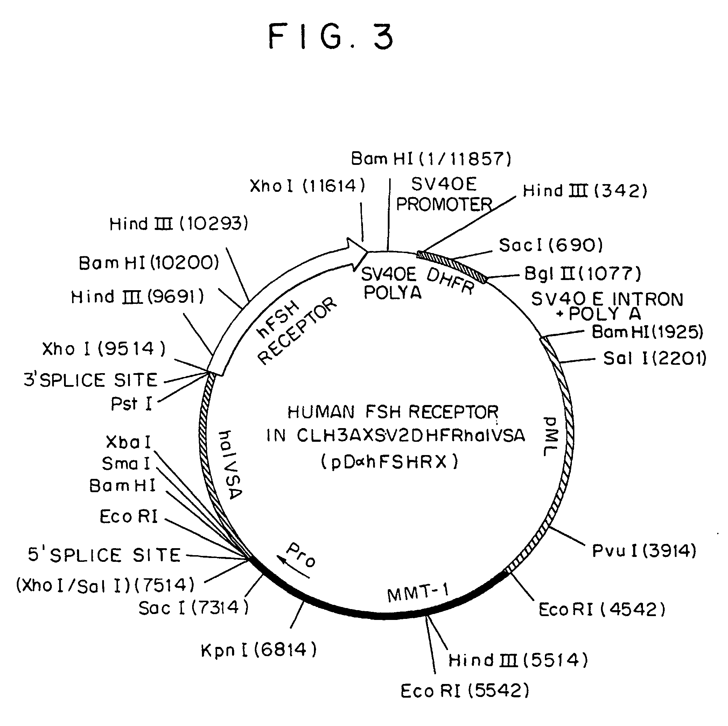

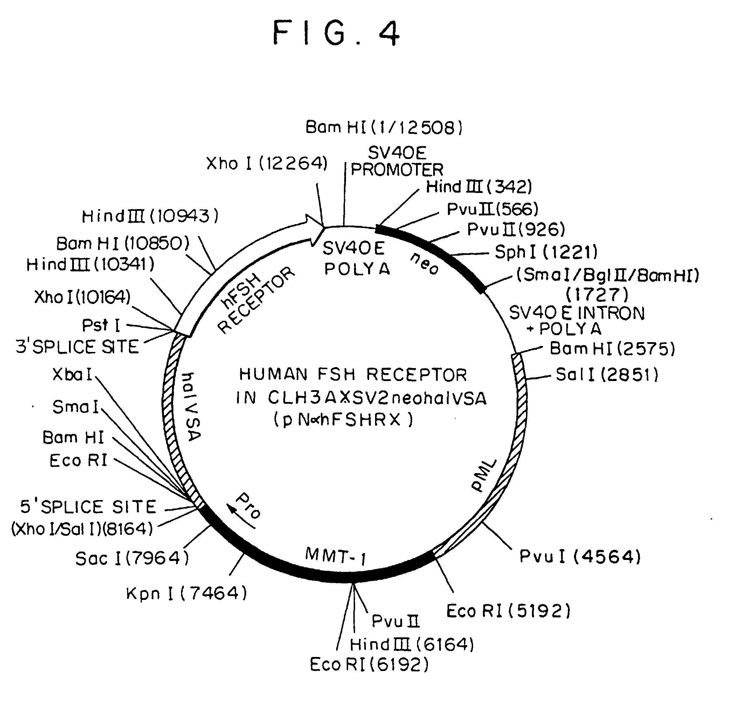

A diagram of the human FSH receptor expression vector used for CHO cell transfections, pDαHFSHRX (human FSH receptor in CLH3AXSV2DHFRhαIVS), is shown in Figure 3 and a diagram of the human FSH receptor expression vector used to stably transfect Y1 cells, pNαHFSHRX (human FSH receptor in CLH3AXSV2NEOhαIVS), is shown in Figure 4. The MMT-I gene sequences (nucleotide numbers 4542-7514 in Figure 3, nucleotide numbers 5192-8164 in Figure 4) which include the promoter region, are the same as those shown upstream of the XhoI site in the construction of CLH3X (Reddy, DNA 6:461, 1987). The human glycoprotein α subunit gene intron A fragment, described in the preceding paragraph, is located between nucleotide numbers 7514 and 9514 in Figure 3, and between nucleotide numbers 8164 and 10164 in Figure 4. Downstream of the XhoI site, the MMT-I introns and polyadenylation signal sequences were removed and replaced with the SV40 early polyadenylation region (nucleotide numbers 11614-11857 in figure 3, nucleotide numbers 12264-12508 in figure 4). The pML region (Lusky, Nature 293:79, 1981) contains the bacterial origin of replication and ampicillin resistance gene from pBR322 (nucleotide numbers 1925-4542 in Figure 3, nucleotide numbers 2575-5192 in Figure 4). The DHFR gene transcription unit (nucleotide numbers 1 to 1925 in Figure 3), which includes the mouse DHFR cDNA and the SV40 early region promoter, small T intron and early region polyadenylation sequences, corresponds to the PvuII-BamHI fragment in pSV2DHFR (Subramani, Mol. Cell. Biol. 1:854, 1981). The PvuII site was converted to a BamHI site prior to insertion of the fragment into the expression vector plasmid. The neo gene transcription unit (nucleotide numbers 1-2575 in figure 4) was assembled by replacing the DHFR cDNA (nucleotide numbers 342-1077 in Figure 3) with a 1385 bp HindIII-SmaI fragment from pSV2-neo (Southern, J. Mol. Appl. Genet. 1:327, 1982). The SmaI site was converted to a BamHI site prior to insertion of the fragment into the expression vector plasmid.

The plasmid pNαhFSHRX can be used to transfect most continuous mammalian cell lines, and was deposited with the ATCC on November 19, 1991, as accession number ATCC68833. The deposit was made in accordance with all of the requirements of the Budapest Treaty. The plasmid pDαhFSHRX is best suited for the transfection of cell lines which lack dihydrofolate reductase (DHFR) activity.

DNA cloning and engineering techniques can be used by those skilled in the art to modify the human FSH receptor DNA expression construction so that FSH-binding fragments are encoded. These modified DNA fragments can be inserted into expression vectors such as those described herein and used to transfect mammalian cells to develop lines which secrete soluble FSH-binding fragments of the human FSH receptor, such as the amino terminal extracellular domain or an FSH-binding fragment thereof.

### Example 3. Development of Mammalian Cell Lines which Stably Express a Functional Human Recombinant FSH Receptor or an FSH-binding fragment or mutant thereof.

Suitable mammalian cell lines for expression of recombinant human FSH and its derivatives include Chinese Hamster Ovary (CHO), mouse adenocarcinoma Y1, rat pituitary GH₃, human breast carcinoma MCF7, and human embryonic kidney 293. In this example, the use of Y1 and CHO cells is described.

Y1 cells are a clonal steroid secreting cell strain initiated from a mouse adrenal cortex tumor (Yasumura, Cancer Res. 26: 529-536, 1966). These cells were obtained from the ATCC cell bank (ATCC CCL 79) and were maintained in culture by growth in Ham's F10 medium supplemented with 15% horse serum (HS), 2.5% FBS, and 1% L-glutamine (Y1 growth medium).

CHO-DUKX cells are a clonal mutant of Chinese hamster ovary cells lacking dihydrofolate reductase activity (Urlaub G. and Chasin, L.A. PNAS 77: 4216-4220, 1980). The cells were maintained in Minimum Essential Medium Alpha (MEM-α) supplemented with 10% FBS and 1% L-glutamine (CHO growth medium).

All cell culturing was done at 37°C in a 5% CO₂, humidified atmosphere.

### Calcium phosphate transfections.

The transfection protocol used was a modification of a published method (Graham, Virology 52: 456, 1973). Approximately 24 hours prior to transfection, cells were plated in 100 mm diameter dishes at a density of 7 X 10⁵ cells/dish (CHO-DUKX) or 1 X 10⁶ cells/dish (Y1). For transfections, 10 µg of the vector plasmid DNA (pDαHFSHRX for CHO-DUKX cells, pNαHFSHRX for Y1 cells) was added to 0.5 ml of transfection buffer. Transfection buffer was prepared by combining 4 grams (g) NaCl, 0.185 g KCl, 0.05 g Na₂HPO₄, 0.5 g dextrose, and 2.5 g HEPES in sterile, distilled water and adjusting the volume to 500 ml, and the pH to 7.5. To the DNA/transfection buffer mixture was added 31 µl of 2 M CaCl₂. The solution was mixed using a vortex mixer and allowed to stand at room temperature for 45 minutes to allow the DNA precipitate to form. The DNA precipitate was layered over the cells after removal of the culture medium. After 20 minutes at room temperature, 5 ml of the appropriate growth medium was added and the cells were cultured for 6 hours. The medium was removed and the cells shocked for 3.5 minutes in 3-4 ml of transfection buffer containing 15% glycerol. The cells were rinsed twice with phosphate buffered saline prior to the addition of 10 ml growth medium. Approximately 48 hours post transfection, the CHO-DUKX cells were subcultured at a 1:10 split ratio and selection medium was added. Y1 cells were allowed to grow for 72 hours post transfection before subculturing at a 1:5 split ratio in selection medium. Selection medium for CHO-DUKX cells consisted of MEM-α without ribonucleosides and deoxyribonucleosides supplemented with 10% dialyzed FBS, 1% L-glutamine, and 0.02 µM methotrexate (MTX). Selection medium for Y1 cells consisted of Y1 growth medium with 80 µg/ml G418. MTX-resistant CHO-DUKX cell colonies and G418-resistant Y1 cell colonies formed after approximately 2 to 3 weeks, and were individually picked and cultured until cell numbers were sufficient for cryopreservation and testing for hormone responsiveness.

### Demonstration of Human Recombinant FSH Receptor Bioactivity in Stably Transfected CHO Cells.

To determine if the engineered human FSH receptor cDNA construct could produce a protein that was biologically functional in stably transfected CHO cells, the MTX-resistant CHO cell colonies were treated with human recombinant FSH and intracellular CAMP levels were measured. For the CAMP assay, the cells were subcultured in 12 well plates at a density of 2.5 X 10⁴ cells/well in CHO cell selection medium. After 72 hours of culture, each well was washed with 1.5 ml of warm growth medium. To each washed well was added 300 µl of serum-free growth medium containing 0.1 mM 3-isobutyl-1-methylxanthine (Sigma). After 15 minutes of culture, human recombinant FSH was added to the medium in each well so that the final concentration was approximately 335 ng (2413 mIU) per ml. After 30 minutes of culture, the assay was terminated by subjecting the cells to four rapid freeze-thaw cycles. A 50 µl aliquot of each cell lysate was transferred to a 1.5 ml tube for determination of protein content. Cold ethanol (300 µl) was added to each remaining lysate sample before transfer to separate 1.5 ml tubes. All lysate samples were centrifuged at 13,000 X g for 15 minutes to remove cell debris. Determination of total soluble protein was done with a protein assay kit purchased from Bio-Rad (catalog number 500-0002) using the protocol supplied by the manufacturer. The ethanol-treated lysate samples were freeze-dried in aliquots of 5-100 µl, and resuspended in 100 µl of the assay buffer provided in the Dupont/NEN Medical Products CAMP assay kit (catalog number NEK-033). The cAMP content of the resuspended lysate samples was determined using the acetylated radioimmunoassay protocol supplied with the kit. The samples were not diluted for the assay. In the majority of the human FSH receptor-transfected CHO cell lines, higher intracellular cAMP levels were measured after FSH stimulation than in the controls (untransfected CHO-DUKX cells and CHO-DUKX cells transfected with CLH3AXSV2DHFRhαIVS). The average response was about 16-fold that of the controls.

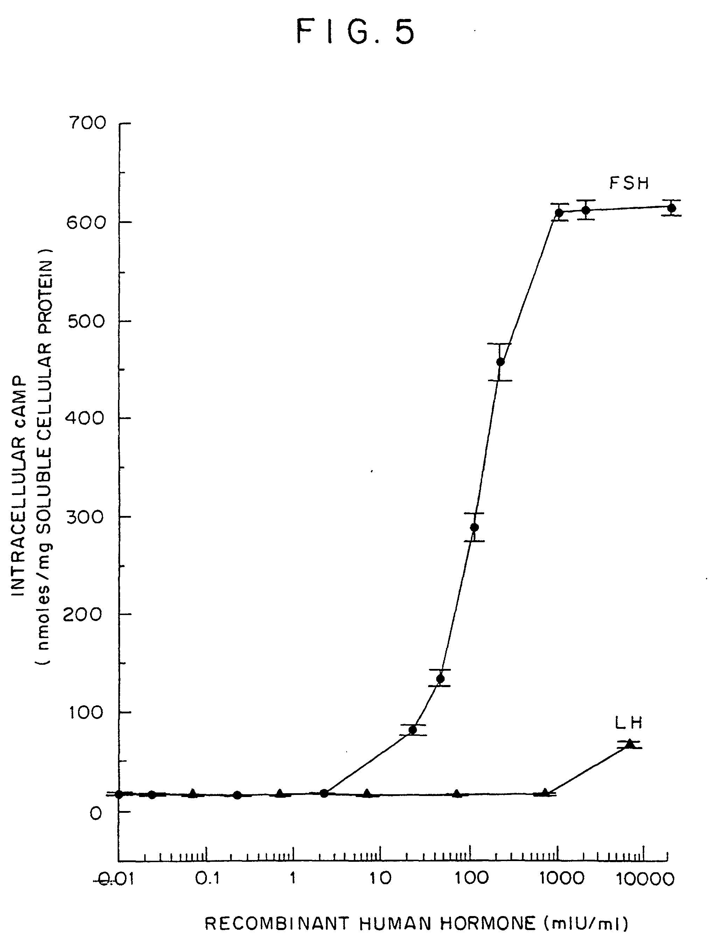

One nonclonal cell line, CHOHFSHR4Q-13, was chosen for a hormone dose response analysis. The procedure used was the same as in the preceding paragraph, except that the cells were treated with either human recombinant FSH or LH in concentrations ranging from 0-10,000 mIU/ml. Each dose was analyzed in triplicate. The amount of intracellular cAMP produced was expressed in nanomoles (nmole) per milligram (mg) of soluble protein and plotted versus FSH or LH concentration to generate dose response curves. The FSH and LH dose response curves are shown in Figure 5. The results show that the CHOHFSHR4Q-13 CHO cell line displayed a saturable increase in intracellular cAMP in response to FSH stimulation but not in response to LH stimulation. Half-maximal FSH stimulation (ED₅₀) occurred at a dose of 144 mIU/ml FSH. The human FSH receptor cDNA therefore can be used to produce a biologically functional human FSH receptor protein in CHO cells, enabling the cells to mediate a specific cAMP response to FSH and not LH.

The human FSH receptor content of the CHOHFSHR4Q-13 line, or a similar line which produces the human recombinant FSH receptor or FSH-binding fragments or mutants thereof, may be increased by exposing the cells to stepwise increases in MIX concentration. This causes amplification of DHFR cDNA copy number along with linked sequences which include the human FSH receptor cDNA, indirectly increasing synthesis of the encoded proteins. This may be done, if necessary, to increase the sensitivity of the bioassay. Also, mammalian cells which express high levels of human FSH receptor or FSH-binding fragments or mutants thereof, can be used for the manufacture of large amounts of human FSH receptor protein for use as therapeutics or in receptor radioimmunoassays.

The CHOHFSHR4Q-13 line, or a similar line, may also be used in an assay for estimating the in vitro bioactivity of pharmaceutical preparations containing FSH, or FSH-like substances. A reporter gene such as luciferase, can be operatively linked to a cAMP response element, and increases in cAMP levels may be indirectly measured by a non-radioactive method, such as bioluminescence, to provide a measurement of FSH bioactivity.

The CHOHFSHR4Q-13 line was deposited with the ATCC on November 19, 1991, as accession number ATCC CRL 10921. The deposit was made in accordance with all the requirements of the Budapest Treaty.

### Demonstration of Recombinant Human FSH Receptor Bioactivity in Stably Transfected Mouse Adrenal Y1 Cells.

To determine if the engineered human FSH receptor cDNA construct could produce a protein that was biologically functional in stably transfected Y1 cells, the G418-resistant Y1 cell colonies were treated with human recombinant FSH and the culture media analyzed for progesterone content. For the progesterone assay, the cells were subcultured in 6 well plates (35 mm well diameter) at a density of 4 X 10⁵ cells/well in Y1 cell selection medium. After two days of culture, the Y1 cell selection medium was removed and replaced with 3 ml Ham's F10 medium supplemented with 5% horse serum, 0.8% fetal bovine serum, 1% L-glutamine, and 80 µg/ml G418. After incubation for one day, the medium was removed and the cells were washed twice with 2 ml of Ham's F10 medium supplemented with 1 mg/ml bovine serum albumin (fraction V), 1% L-glutamine, and 80 µg/ml G418 (assay medium). At this time the average cell number per well was estimated for each cell line by removing and counting cells from three test wells. The cells were then cultured for 4 hours with 1 ml assay medium containing 100 mIU/ml human recombinant FSH. After incubation, the culture dishes were placed on ice while the culture medium from each well was transferred into separate glass 12 X 75 test tubes. The tubes containing the culture media were placed in a boiling water bath for 10 minutes, then centrifuged at 1100 X g at 4°C. The supernatants were transferred to clean 12 X 75 glass test tubes and stored at -20°C overnight. Progesterone levels were measured using the Serono Diagnostics Progesterone MAIA, product number 12274, distributed by Ciba Corning, Medfield, MA. The assay was done according to the instructions provided by the manufacturer, except that the culture medium samples and the progesterone for the standard curve were diluted in a solution of phosphate buffered saline containing 0.1% bovine serum albumin and 0.6% sodium azide, instead of the dilution buffer provided in the kit. The progesterone for the standard curve was obtained from Cal Biochem (catalog number 5341), and was diluted to a concentration of 15 µg/ml in 100% ethanol prior to use. The stock solution was stored at -70°C. The final concentrations of the progesterone standard curve dilutions were the same as those recommended in the assay kit. The diluted progesterone standards were stable at 4°C for one week.

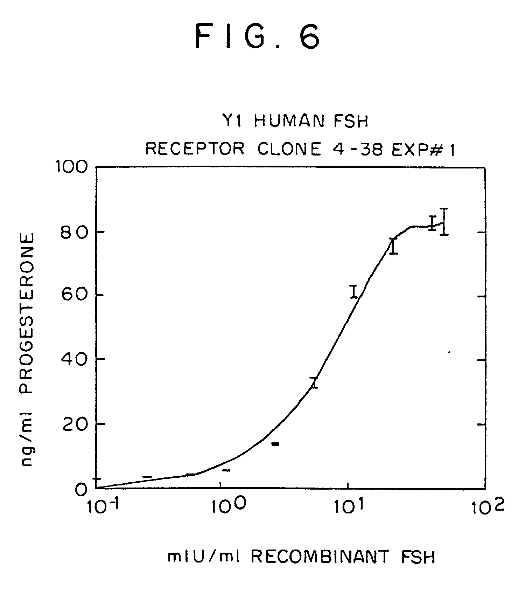

Several FSH-treated Y1 lines, stably transfected with the human FSH receptor, secreted increased levels of progesterone relative to the controls (the same line not treated with FSH, untransfected Y1 cells, Y1 cells transfected with CLH3AXSV2NEOhαIVS). One nonclonal line, Y1HFSHR4-38, was chosen for hormone dose response analyses. The procedure used was the same as that described in the preceding paragraph except that the cells were treated with a range of FSH concentrations, 0-100 mIU/ml. Each dose was done in duplicate. The amount of progesterone produced by the FSH-stimulated Y1 cells was normalized to 1 X 10⁶ cells and plotted versus FSH concentration to generate a dose response curve. The dose response curve is shown in Figure 6. In this particular experiment, a 25-fold increase in progesterone activity was observed at a dose of 20 mIU/ml human recombinant FSH. The linear range was from 2.5 to 20 mIU/ml FSH and the ED₅₀ was 6.4 mIU/ml FSH. These results demonstrate that FSH dose dependent increases in progesterone secretion were observed in Y1 cells stably transfected with the human recombinant FSH receptor. The human FSH receptor is therefore functionally active when expressed in mouse adrenal Y1 cells.

Cell populations derived from the CHOHFSHR4Q-13 or Y1HFSHR4-38 cell lines, or similar cell lines, can be used to develop sensitive in vitro bioassays for FSH and FSH-like substances. Other FSH in vitro bioassays which are currently used, such as the granulosa cell aromatase bioassay and the Sertoli cell aromatase bioassay, require the establishment of primary cell cultures from rats each time an assay is performed. For the new Y1 cell and CHO cell bioassays, stably transformed continuous cell lines are used. This enables the practice of in vitro bioassay methods superior to the current methods in simplicity, accuracy, and consistency.

### SEQUENCE LISTING

### INFORMATION FOR SEQ ID NO: 1

### SEQUENCE CHARACTERISTICS

(A) LENGTH: 2179
(B) TYPE: Nucleic acid
(C) STRANDEDNESS: Double
(D) TOPOLOGY: Linear

### MOLECULE TYPE: cDNA to mRNA

### ORIGINAL SOURCE:

(A) ORGANISM: Homo sapiens
(F) TISSUE TYPE: Testis

### IMMEDIATE SOURCE:

(A) LIBRARY: λgt11 cDNA library, ClonTech #HL1010b
(B) CLONE: pHFSHR11-11, pHFSHR15-6

### FEATURE:

(A) NAME/KEY: protein coding region
(B) LOCATION: 75 to 2159

### SEQUENCE DESCRIPTION: SEQ ID NO:1:

TGTGGAGCTT CTGAGATCTG TGGAGGTTTT TCTCTGCAAA TGCAGGAAGA AATCAGGTGG 60

ATGGATGCAT AATT ATG GCC CTG CTC CTG GTC TCT TTG CTG GCA TTC CTG 110

-15 -10

AGC TTG GGC TCA GGA TGT CAT CAT CGG ATC TGT CAC TGC TCT AAC AGG 158

-5 1 5 10

GTT TTT CTC TGC CAA GAG AGC AAG GTG ACA GAG ATT CCT TCT GAC CTC 206

15 20 25

CCG AGG AAT GCC ATT GAA CTG AGG TTT GTC CTC ACC AAG CTT CGA GTC 254

30 35 40

ATC CAA AAA GGT GCA TTT TCA GGA TTT GGG GAC CTG GAG AAA ATA GAG 302

45 50 55

ATC TCT CAG AAT GAT GTC TTG GAG GTG ATA GAG GCA GAT GTG TTC TCC 350

60 65 70 75

AAC CTT CCC AAA TTA CAT GAA ATT AGA ATT GAA AAG GCC AAC AAC CTG 398

80 85 90

CTC TAC ATC AAC CCT GAG GCC TTC CAG AAC CTT CCC AAC CTT CAA TAT 446

95 100 105

CTG TTA ATA TCC AAC ACA GGT ATT AAG CAC CTT CCA GAT GTT CAC AAG 494

110 115 120

ATT CAT TCT CTC CAA AAA GTT TTA CTT GAC ATT CAA GAT AAC ATA AAC 542

125 130 135

ATC CAC ACA ATT GAA AGA AAT TCT TTC GTG GGG CTG AGC TTT GAA AGT 590

140 145 150 155

GTG ATT CTA TGG CTG AAT AAG AAT GGG ATT CAA GAA ATA CAC AAC TGT 638

160 165 170

GCA TTC AAT GGA ACC CAA CTA GAT GAG CTG AAT CTA AGC GAT AAT AAT 686

175 180 185

AAT TTA GAA GAA TTG CCT AAT GAT GTT TTC CAC GGA GCC TCT GGA CCA 734

190 195 200

GTC ATT CTA GAT ATT TCA AGA ACA AGG ATC CAT TCC CTG CCT AGC TAT 782

205 210 215

GGC TTA GAA AAT CTT AAG AAG CTG AGG GCC AGG TCG ACT TAC AAC TTA 830

220 225 230 235

AAA AAG CTG CCT ACT CTG GAA AAG CTT GTC GCC CTC ATG GAA GCC AGC 878

240 245 250

CTC ACC TAT CCC AGC CAT TGC TGT GCC TTT GCA AAC TGG AGA CGG CAA 926

255 260 265

ATC TCT GAG CTT CAT CCA ATT TGC AAC AAA TCT ATT TTA AGG CAA GAA 974

270 275 280

GTT GAT TAT ATG ACT CAG ACT AGG GGT CAG AGA TCC TCT CTG GCA GAA 1022

285 290 295

GAC AAT GAG TCC AGC TAC AGC AGA GGA TTT GAC ATG ACG TAC ACT GAG 1070

300 305 310 315

TTT GAC TAT GAC TTA TGC AAT GAA GTG GTT GAC GTG ACC TGC TCC CCT 1118

320 325 330

AAG CCA GAT GCA TTC AAC CCA TGT GAA GAT ATC ATG GGG TAC AAC ATC 1166

335 340 345

CTC AGA GTC CTG ATA TGG TTT ATC AGC ATC CTG GCC ATC ACT GGG AAC 1214

350 355 360

ATC ATA GTG CTA GTG ATC CTA ACT ACC AGC CAA TAT AAA CTC ACA GTC 1262

365 370 375

CCC AGG TTC CTT ATG TGC AAC CTG GCC TTT GCT GAT CTC TGC ATT GGA 1310

380 385 390 395

ATC TAC CTG CTG CTC ATT GCA TCA GTT GAT ATC CAT ACC AAG AGC CAA 1358

400 405 410

TAT CAC AAC TAT GCC ATT GAC TGG CAA ACT GGG GCA GGC TGT GAT GCT 1406

415 420 425

GCT GGC TTT TTC ACT GTC TTT GCC AGT GAG CTG TCA GTC TAC ACT CTG 1454

430 435 440

ACA GCT ATC ACC TTG GAA AGA TGG CAT ACC ATC ACG CAT GCC ATG CAG 1502

445 450 455

CTG GAC TGC AAG GTG CAG CTC CGC CAT GCT GCC AGT GTC ATG GTG ATG 1550

460 465 470 475

GGC TGG ATT TTT GCT TTT GCA GCT GCC CTC TTT CCC ATC TTT GGC ATC 1598

480 485 490

AGC AGC TAC ATG AAG GTG AGC ATC TGC CTG CCC ATG GAT ATT GAC AGC 1646

495 500 505

CCT TTG TCA CAG CTG TAT GTC ATG TCC CTC CTT GTG CTC AAT GTC CTG 1694

510 515 520

GCC TTT GTG GTC ATC TGT GGC TGC TAT ATC CAC ATC TAC CTC ACA GTG 1742

525 530 535

CGG AAC CCC AAC ATC GTG TCC TCC TCT AGT GAC ACC AGG ATC GCC AAG 1790

540 545 550 555

CGC ATG GCC ATG CTC ATC TTC ACT GAC TTC CTC TGC ATG GCA CCC ATT 1838

560 565 570

TCT TTC TTT GCC ATT TCT GCC TCC CTC AAG GTG CCC CTC ATC ACT GTG 1886

575 580 585

TCC AAA GCA AAG ATT CTG CTG GTT CTG TTT CAC CCC ATC AAC TCC TGT 1934

590 595 600

GCC AAC CCC TTC CTC TAT GCC ATC TTT ACC AAA AAC TTT CGC AGA GAT 1982

605 610 615

TTC TTC ATT CTG CTG AGC AAG TGT GGC TGC TAT GAA ATG CAA GCC CAA 2030

620 625 630 635

ATT TAT AGG ACA GAA ACT TCA TCC ACT GTC CAC AAC ACC CAT CCA AGG 2078

640 645 650

AAT GGC CAC TGC TCT TCA GCT CCC AGA GTC ACC AAT GGT TCC ACT TAC 2126

655 660 665

ATA CTT GTC CCT CTA AGT CAT TTA GCC CAA AAC TAAAACACAA TGTGAAAATG 2179

670 675

### INFORMATION FOR SEQ ID NO: 2

### SEQUENCE CHARACTERISTICS

(A) LENGTH: 695
(B) TYPE: Amino acid
(D) TOPOLOGY: Linear

### MOLECULE TYPE: protein

### FEATURE:

(A) NAME/KEY: signal sequence
(B) LOCATION: -17 to -1
(C) IDENTIFICATION METHOD: hydrophobic

### FEATURE:

(A) NAME/KEY: putative amino-terminal extracellular domain
(B) LOCATION: 1 to 349
(C) IDENTIFICATION METHOD: similarity with other dimeric glycoprotein receptor extracellular domains, hydrophilic

### FEATURE:

(A) NAME/KEY: transmembrane domain
(B) LOCATION: 350 to 613
(C) IDENTIFICATION METHOD: similarity to other G protein-coupled receptor transmembrane domains

### FEATURE:

(A) NAME/KEY: putative transmembrane region I
(B) LOCATION: 350 to 370
(C) IDENTIFICATION METHOD: similarity to other G protein-coupled receptor transmembrane regions, hydrophobic, about 20-23 amino acids in length

### FEATURE:

(A) NAME/KEY: putative transmembrane region II
(B) LOCATION: 382 to 404
(C) IDENTIFICATION METHOD: similarity to other G protein-coupled receptor transmembrane regions, hydrophobic, about 20-23 amino acids in length

### FEATURE:

(A) NAME/KEY: putative transmembrane region III
(B) LOCATION: 427 to 448
(C) IDENTIFICATION METHOD: similarity to other G protein-coupled receptor transmembrane regions, hydrophobic, about 20-23 amino acids in length

### FEATURE:

(A) NAME/KEY: putative transmembrane region IV
(B) LOCATION: 469 to 491
(C) IDENTIFICATION METHOD: similarity to other G protein-coupled receptor transmembrane regions, hydrophobic, about 20-23 amino acids in length

### FEATURE:

(A) NAME/KEY: putative transmembrane region V
(B) LOCATION: 512 to 533
(C) IDENTIFICATION METHOD: similarity to other G protein-coupled receptor transmembrane regions, hydrophobic, about 20-23 amino acids in length

### FEATURE:

(A) NAME/KEY: putative transmembrane region VI
(B) LOCATION: 557 to 580
(C) IDENTIFICATION METHOD: similarity to other G protein-coupled receptor transmembrane regions, hydrophobic, about 20-23 amino acids in length

### FEATURE:

(A) NAME/KEY: putative transmembrane region VII
(B) LOCATION: 592 to 613
(C) IDENTIFICATION METHOD: similarity to other G protein-coupled receptor transmembrane regions, hydrophobic, about 20-23 amino acids in length

### FEATURE:

(A) NAME/KEY: putative carboxy-terminal intracellular domain
(B) LOCATION: 614 to 678

### SEQUENCE DESCRIPTION: SEQ ID NO:2:


-15 -10 -5


1               5                   10                  15


            20                  25                  30


        35                  40                  45


    50                  55                  60


65 70 75


80 85 90 95


            100                 105                 110


        115                 120                 125


    130                 135                 140


145 150 155


160 165 170 175


            180                 185                 190


        195                 200                 205


    210                 215                 220


225 230 235


240 245 250 255


            260                 265                 270


            275                 280                 285


        290                 295                 300


    305                 310                 315


320                 325                 330                 335


                340                 345                 350


            355                 360                 365


        370                 375                 380


    385                 390                 395


400                 405                 410                 415


                420                 425                 430


            435                 440                 445


        450                 455                 460


    465                 470                 475


480                 485                 490                 495


                500                 505                 510


            515                 520                 525


        530                 535                 540


    545                 550                 555


560                 565                 570                 575


                580                 585                 590


            595                 600                 605


        610                 615                 620


    625                 630                 635


640                 645                 650                 655


                660                 665                 670


            675

## Claims (Claims for the following Contracting State(s): AT-BE-CH-DE-DK-FR-GB-IT-LU-MC-NL-SE)

1. A pure polypeptide capable of recognising and selectively binding human follicle stimulating hormone (FSH), and which produces no immunological response in humans, consisting of:
(a) the human FSH receptor having amino acid sequence 1-678 shown in SEQ ID NO:2;
(b) a mutein which retains at least 95% identity with said sequence of (a);
(c) a polypeptide which includes the amino-terminal extracellular portion of the human FSH receptor, including the amino acid sequence 1-349 shown in SEQ ID NO:2;
(d) a polypeptide which includes a mutein which mutein retains at least 95% identity with the amino acid sequence 1-349 shown in SEQ. ID. NO 2;
(e) a polypeptide which includes a fragment of the extracellular portion of the human FSH receptor of (a) which fragment retains the FSH-binding characteristics as the complete extracellular portion; or
(f) a polypeptide which includes a mutein of said fragment of (e), which mutein retains at least 95% identity with said fragment and which retains the same FSH-binding characteristics as the complete extracellular portion of the human FSH receptor, which complete extracellular portion includes the amino acid sequence 1-349 shown in SEQ ID NO. 2.

2. A polypeptide capable of binding human FSH, in accordance with claim 1, consisting of the amino acid sequence 1-678 shown in SEQ ID NO:2 or a mutein which retains at least 95% identity with said sequence.

3. A polypeptide capable of binding human FSH, in accordance with claim 1, consisting of the amino acid sequence 1-613 shown in SEQ ID NO:2 or a mutein which retains at least 95% identity with said sequence.

4. A polypeptide capable of binding human FSH, in accordance with claim 1, consisting of the amino acid sequence 1-349 shown in SEQ ID NO:2 or a mutein which retains at least 95% identity with said sequence.

5. Mutein form of the polypeptide according to claim 1 (a), (c) or (e), in which that portion corresponding to all or part of the natural human FSH receptor includes conservative substitutions in one to ten amino acid residues, the location and nature of said substitutions being selected so as to retain the same FSH-binding characteristics of the receptor or fragment thereof which is unmodified.

6. Isolated DNA encoding a polypeptide in accordance with any one of claims 1-5.

7. The isolated DNA of claim 6 comprising the nucleotide sequence shown in SEQ ID NO:1.

8. A recombinant expression vector comprising the DNA of claims 6 or 7.

9. A cell transfected with the expression vector of claim 8.

10. The cell of claim 9 which is a mammalian cell.

11. A method of producing a polypeptide of claims 1-6, which comprises culturing the cell of claims 9 or 10 in a nutrient medium and recovering the receptor therefrom.

12. A pharmaceutical composition comprising a polypeptide in accordance with any one of claims 1-5 and a pharmaceutically acceptable carrier.

13. Pharmaceutical composition according to claim 12, for use in reducing endogenous FSH-bioactivity in a patient.

14. Pharmaceutical composition according to claim 12 for use in preventing follicle growth and maturation in female patient.

15. Pharmaceutical composition according to claim 12 for use in preventing spermatogenesis in a male patient.

16. Method of assaying human FSH, *ex vivo*, using a FSH receptor, **characterized in that** a polypeptide according to any one of claims 1-5 is used as FSH receptor.

## Claims (Claims for the following Contracting State(s): ES, GR)

1. Method of producing a pure polypeptide capable of recognising and selectively binding human follicle stimulating hormone (FSH), and which produces no immunological response in humans, the polypeptide consisting of:
(a) the human FSH receptor having amino acid sequence 1-678 shown in SEQ ID NO:2;
(b) a mutein which retains at least 95% identity with said sequence of (a);
(c) a polypeptide which includes the amino-terminal extracellular portion of the human FSH receptor, including the amino acid sequence 1-349 shown in SEQ ID NO:2;
(d) a polypeptide which includes a mutein which mutein retains at least 95% identity with the amino acid sequence 1-349 shown in SEQ ID NO 2;
(e) a polypeptide which includes a fragment of the extracellular portion of the human FSH receptor of (a) which fragment retains the FSH-binding characteristics as the complete extracellular portion; or
(f) a polypeptide which includes a mutein of said fragment of (e), which mutein retains at least 95% identity with said fragment and which retains the same FSH-binding characteristics as the complete extracellular portion of the human FSH receptor, which complete extracellular portion includes the amino acid sequence 1-349 shown in SEQ ID NO: 2, comprising the steps of culturing cells transfected with a recombinant expression vector, the vector comprising DNA encoding the said polypeptide, in a nutrient medium and recovering the polypeptide therefrom.

2. Method according to claim 1, wherein the polypeptide capable of binding human FSH consists of the amino acid sequence 1-678 shown in SEQ ID NO:2 or a mutein which retains at least 95% identity with said sequence.

3. Method according to claim 1, wherein the polypeptide capable of binding human FSH consists of the amino acid sequence 1-613 shown in SEQ ID NO:2 or a mutein which retains at least 95% identity with said sequence.

4. Method according to claim 1, wherein the polypeptide capable of binding human FSH, in accordance with claim 1, consisting of the amino acid sequence 1-349 shown in SEQ ID NO:2 or a mutein which retains at least 95% identity with said sequence.

5. Method according to claim 1, wherein the polypeptide is a mutein form of the polypeptide according to claim 1 (a), (c) or (e), in which that portion corresponding to all or part of the natural human FSH receptor includes conservative substitutions in one to ten amino acid residues, the location and nature of said substitutions being selected so as to retain the same FSH-binding characteristics of the receptor or fragment thereof which is unmodified.

6. Method for the preparation of a mammation cell, harboring the expression vector comprising DNA encoding a polypeptide as defined in claims 1-5, comprising the steps of transfecting the cell with the said expression vector, and culturing the transfected cells.

7. Method for the preparation of a pharmaceutical composition comprising a polypeptide as defined in any one of claims 1-5, comprising the step of formulating the polypeptide with a pharmaceutically acceptable carrier.

8. Use of a polypeptide as defined in any one of claims 1-5 for the preparation of a pharmaceutical composition for reducing endogenous FSH-bioactivity in a patient.

9. Use of a polypeptide as defined in any one of claims 1-5 for the preparation of a pharmaceutical composition for preventing follicle growth and maturation in a female patient.

10. Use of a polypeptide as defined in any one of claims 1-5 for the preparation of a pharmaceutical composition for preventing spermatogenesis in a male patient.

11. Method of assaying human FSH, *ex vivo,* using a FSH receptor, **characterized in that** a polypeptide as defined in any one of claims 1-5 is used as FSH receptor.

## Patentansprüche (Patentansprüche für folgende(n) Vertragsstaat(en): AT-BE-CH-DE-DK-FR-GB-IT-LU-MC-NL-SE)

1. Ein reines Polypeptid, das in der Lage ist, menschliches follikelstimulierendes Hormon (FSH) zu erkennen und selektiv zu binden, und welches keine immunologische Antwort beim Menschen hervorruft, bestehend aus:
a. dem menschlichen FSH-Rezeptor mit der Aminosäuresequenz 1 bis 678, dargestellt in SEQ ID NO:2;
b. einem Mutein, welches mindestens 95% Identität mit der Sequenz aus (a) aufweist;
c. einem Polypeptid, enthaltend den aminoterminalen extrazellulären Anteil des menschlichen FSH-Rezeptors, einschließlich der Aminosäuresequenz 1 bis 349, dargestellt in SEQ ID NO:2;
d. einem Polypeptid, enthaltend ein Mutein, wobei das Mutein mindestens 95% Identität mit der Aminosäuresequenz 1 bis 349, dargestellt in SEQ ID NO:2, aufweist;
e. ein Polypeptid, enthaltend ein Fragment des extrazellulären Anteils des menschlichen FSH-Rezeptors gemäß (a), wobei das Fragment die FSH-Bindungscharakteristiken des vollständigen extrazellulären Anteil aufweist; oder
f. ein Polypeptid, enthaltend ein Mutein des Fragments gemäß (e), wobei das Mutein mindestens 95% Identität mit dem Fragment aufweist, und welches die gleichen FSH-Bindungscharakteristiken wie der vollständige extrazelluläre Anteil aufweist, wobei der vollständige extrazelluläre Anteil die Aminosäuresequenz 1 bis 349, dargestellt in SEQ ID NO:2, enthält.

2. Ein Polypeptid, welches in der Lage ist, menschliches FSH zu binden, gemäß Anspruch 1, bestehend aus der Aminosäuresequenz 1 bis 678, dargestellt in SEQ ID NO:2, oder ein Mutein, welches mindestens 95% Identität mit dieser Sequenz aufweist.

3. Ein Polypeptid, das in der Lage ist, menschliches FSH zu binden, gemäß Anspruch 1, bestehend aus der Aminosäuresequenz 1 bis 613, dargestellt in SEQ ID NO:2, oder ein Mutein, welches mindestens 95% Identität mit dieser Sequenz aufweist.

4. Ein Polypeptid, das in der Lage ist, menschliches FSH zu binden, gemäß Anspruch 1, bestehend aus der Aminosäure-sequenz 1 bis 349, dargestellt in SEQ ID NO:2, oder ein Mutein, welches mindestens 95% Identität mit dieser Sequenz aufweist.

5. Eine Muteinform des Polypeptids nach Anspruch 1 (a), (c) oder (e), in welcher der Anteil, der dem ganzen oder einem Teil des natürlichen menschlichen FSH-Rezeptors entspricht, konservative Substitutionen in ein bis zehn Aminosäureresten beinhaltet, wobei die Position und Art der Substitutionen so gewählt ist, dass die gleichen FSH-Bindungscharakteristiken des nicht modifizierten Rezeptors oder Fragments davon erhalten bleiben.

6. Isolierte DNA, kodierend ein Polypeptid nach einem der Ansprüche 1 bis 5.

7. Isolierte DNA nach Anspruch 6, enthaltend die in SEQ ID NO:1 dargestellte Nukleotidsequenz.

8. Rekombinanter Expressionsvektor, enthaltend die DNA nach Anspruch 6 oder 7.

9. Zelle, die mit dem Expressionsvektor nach Anspruch 8 transfiziert ist.

10. Zelle nach Anspruch 9, die eine Säugetierzelle darstellt.

11. Verfahren zur Herstellung eines Polypeptids nach Anspruch 1 bis 6, welches umfasst: Kultivierung der Zelle nach Anspruch 9 oder 10 in einem Nährmedium und Gewinnung des Rezeptors daraus.

12. Pharmazeutische Zusammensetzung, enthaltend ein Polypeptid nach einem Ansprüche 1 bis 5 und einen pharmazeutische akzeptablen Träger.

13. Pharmazeutische Zusammensetzung nach Anspruch 12 zur Verwendung bei der Verminderung der endogenen FSH-Bioaktivität bei einem Patienten.

14. Pharmazeutische Zusammensetzung nach Anspruch 12 zur Verwendung bei der Verhinderung (Prävention) von Follikelwachstum und -maturation bei einem weiblichen Patienten.

15. Pharmazeutische Zusammensetzung nach Anspruch 12 zur Verwendung bei der Verhinderung (Prävention) der Spermatogenese bei einem männlichen Patienten.

16. Verfahren zur Bestimmung von menschlichem FSH *ex vivo* unter Verwendung eines FSH-Rezeptors, **dadurch gekennzeichnet, dass** das Polypeptid nach einem der Ansprüche 1 bis 5 als FSH-Rezeptor verwendet wird.

## Patentansprüche (Patentansprüche für folgende(n) Vertragsstaat(en): ES und GR)

1. Verfahren zur Herstellung eines reinen Polypeptids, das in der Lage ist, menschliches follikelstimulierendes Hormon (FSH) zu erkennen und selektiv zu binden, und welches keine immunologische Antwort beim Menschen hervorruft, wobei das Polypeptid besteht aus:
a. dem menschlichen FSH-Rezeptor mit der Aminosäuresequenz 1 bis 678, dargestellt in SEQ ID NO:2;
b. einem Mutein, welches mindestens 95% Identität mit der Sequenz aus (a) aufweist;
c. einem Polypeptid, enthaltend den aminoterminalen extrazellulären Anteil des menschlichen FSH-Rezeptors, einschließlich der Aminosäuresequenz 1 bis 349, dargestellt in SEQ ID NO:2;
d. einem Polypeptid, enthaltend ein Mutein, wobei das Mutein mindestens 95% Identität mit der Aminosäuresequenz 1 bis 349, dargestellt in SEQ ID NO:2, aufweist;
e. ein Polypeptid, enthaltend ein Fragment des extrazellulären Anteils des menschlichen FSH-Rezeptors gemäß (a), wobei das Fragment die FSH-Bindungscharakteristiken des vollständigen extrazellulären Anteil aufweist; oder
f. ein Polypeptid, enthaltend ein Mutein des Fragments gemäß (e), wobei das Mutein mindestens 95% Identität mit dem Fragment aufweist, und welches die gleichen FSH-Bindungscharakteristiken wie der vollständige extrazelluläre Anteil aufweist, wobei der vollständige extrazelluläre Anteil die Aminosäuresequenz 1 bis 349, dargestellt in SEQ ID NO:2, enthält, umfassend die [nachstehenden] Schritte: Kultivierung der mit einem rekombinanten Expressionsvektor transfizierten Zellen, wobei der Vektor eine DNA enthält, die für das Polypeptid kodiert, in einem Nährmedium und Wiedergewinnung des Polypeptids daraus.

2. Verfahren gemäß Anspruch 1, wobei das Polypeptid, welches in der Lage ist, menschliches FSH zu binden, aus der Aminosäuresequenz 1 bis 678 besteht, dargestellt in SEQ ID NO:2, oder einem Mutein, welches mindestens 95% Identität mit dieser Sequenz aufweist.

3. Verfahren gemäß Anspruch 1, wobei das Polypeptid, das in der Lage ist, menschliches FSH zu binden, aus der Aminosäuresequenz 1 bis 613 besteht, dargestellt in SEQ ID NO:2, oder einem Mutein, welches mindestens 95% Identität mit dieser Sequenz aufweist.

4. Verfahren gemäß Anspruch 1, wobei das Polypeptid, das in der Lage ist, menschliches FSH zu binden, gemäß Anspruch 1, aus der Aminosäuresequenz 1 bis 349 besteht, dargestellt in SEQ ID NO:2, oder einem Mutein, welches mindestens 95% Identität mit dieser Sequenz aufweist.

5. Verfahren gemäß Anspruch 1, wobei das Polypeptid eine Muteinform des Polypeptids nach Anspruch 1 (a), (c) oder (e) ist, in welcher der Anteil, der dem ganzen oder einem Teil des natürlichen menschlichen FSH-Rezeptors entspricht, konservative Substitutionen in ein bis zehn Aminosäureresten beinhaltet, wobei die Position und Art der Substitutionen so gewählt ist, dass die gleichen FSH-Bindungscharakteristiken des nicht modifizierten Rezeptors oder Fragments davon erhalten bleiben.

6. Verfahren zur Herstellung einer Säugetierzelle mit dem Expressionsvektor, enthaltend eine ein wie in den Ansprüchen 1 bis 5 definiertes Polypeptid kodierende DNA, umfassend die [nachstehenden] Schritte: Transfizieren der Zelle mit dem Expressionsvektor und Kultivierung der transfizierten Zellen.

7. Verfahren zur Herstellung einer ein Polypeptid nach einem der Ansprüche 1 bis 5 enthaltenden pharmazeutischen Zusammensetzung, welches den Schritt der Formulierung des Polypeptids mit einem pharmazeutisch akzeptablen Träger umfasst.

8. Verwendung eines Polypeptids nach einem der Ansprüche 1 bis 5 zur Herstellung einer pharmazeutischen Zusammensetzung zur Verminderung der endogenen FSH-Bioaktivität bei einem Patienten.

9. Verwendung eines Polypeptids nach einem der Ansprüche 1 bis 5 zur Herstellung einer pharmazeutischen Zusammensetzung zur Verhinderung (Prävention) von Follikelwachstum und -maturation bei einem weiblichen Patienten.

10. Verwendung eines Polypeptids nach einem der Ansprüche 1 bis 5 zur Herstellung einer pharmazeutischen Zusammensetzung zur Verhinderung (Prävention) der Spermatogenese bei einem männlichen Patienten.

11. Verfahren zur Bestimmung von menschlichem FSH *ex vivo* unter Verwendung eines FSH-Rezeptors, **dadurch gekennzeichnet, dass** das Polypeptid nach einem der Ansprüche 1 bis 5 als FSH-Rezeptor verwendet wird.

## Revendications (Revendications pour l'(les) Etat(s) contractant(s) suivant(s): AT-BE-CH-DE-DK-FR-GB-IT-LU-MC-NL-SE)

1. Polypeptide pur capable de reconnaître et de se lier sélectivement à l'hormone folliculo-stimulante (FSH) humaine et qui ne produise pas de réponse immunologique chez l'homme, consistant en :
(a) le récepteur de la FSH humaine ayant la séquence d'acides aminés 1-678 présentée dans SEQ ID N° 2 ;
(b) une mutéine qui conserve au moins 95 % d'identité avec ladite séquence de (a) ;
(c) un polypeptide qui inclut la partie extracellulaire amino-terminale du récepteur de la FSH humaine, incluant la séquence d'acides aminés 1-349 présentée dans SEQ ID N°2 ;
(d) un polypeptide qui inclut une mutéine ladite mutéine conservant au moins 95 % d'identité avec la séquence d'acides aminés 1-349 présentée dans SEQ ID N° 2 ;
(e) un polypeptide qui inclut un fragment de la partie extracellulaire du récepteur de la FSH humaine de (a), ledit fragment conservant les caractéristiques de liaison à la FSH de la partie extracellulaire complète ; ou
(f) un polypeptide qui inclut une mutéine dudit fragment de (e), ladite mutéine conservant au moins 95 % d'identité avec ledit fragment et conservant les mêmes caractéristiques de liaison à la FSH que la partie extracellulaire complète du récepteur de la FSH humaine, ladite partie extracellulaire complète incluant la séquence d'acides aminés 1-349 présentée dans SEQ ID N° 2.

2. Polypeptide capable de se lier à la FSH humaine, selon la revendication 1, consistant en la séquence d'acides aminés 1-678 présentée dans SEQ ID N° 2 ou une mutéine qui conserve au moins 95 % d'identité avec ladite séquence.

3. Polypeptide capable de se lier à la FSH humaine, selon la revendication 1, consistant en la séquence d'acides aminés 1-613 présentée dans SEQ ID N° 2 ou une mutéine qui conserve au moins 95 % d'identité avec ladite séquence.

4. Polypeptide capable de se lier à la FSH humaine, selon la revendication 1, consistant en la séquence d'acides aminés 1-349 présentée dans SEQ ID N° 2 ou une mutéine qui conserve au moins 95 % d'identité avec ladite séquence.

5. Forme mutéine du polypeptide selon la revendication 1 (a), (c) ou (e), dans lequel la partie correspondant à tout ou partie du récepteur naturel de la FSH humaine inclut des substitutions conservatives dans un à dix résidus d'acides aminés, l'emplacement et la nature desdites substitutions étant sélectionnés pour conserver les mêmes caractéristiques de liaison de la FSH du récepteur ou du fragment de récepteur qui n'est pas modifié.

6. ADN isolé codant pour un polypeptide selon l'une quelconque des revendications 1-5.

7. ADN isolé de la revendication 6 comprenant la séquence de nucléotides de SEQ ID N° 1.

8. Vecteur d'expression recombinant comprenant l'ADN des revendications 6 ou 7.

9. Cellule transfectée avec le vecteur d'expression de la revendication 8.

10. Cellule de la revendication 9 qui est une cellule de mammifère.

11. Méthode de fabrication d'un polypeptide selon les revendications 1 à 6, qui consiste à mettre en culture la cellule de la revendication 9 ou 10 dans un milieu nutritif et en récupérant le récepteur de cette cellule.

12. Composition pharmaceutique comprenant un polypeptide selon l'une quelconque des revendications 1 à 5 et un porteur pharmaceutiquement acceptable.

13. Composition pharmaceutique selon la revendication 12 à utiliser pour réduire la bioactivité de la FSH endogène chez un patient.

14. Composition pharmaceutique selon la revendication 12 à utiliser pour empêcher la croissance et la maturation des follicules chez une patiente.

15. Composition pharmaceutique selon la revendication 12 à utiliser pour empêcher la spermatogenèse chez un patient.

16. Méthode de dosage de la FSH humaine *ex vivo* utilisant un récepteur de la FSH, **caractérisée en ce qu'**un polypeptide selon l'une quelconque des revendications 1 à 5 est utilisé comme récepteur de la FSH.

## Revendications (Revendications pour l'(les) Etat(s) contractant(s) suivant(s): GR, ES)

1. Méthode de fabrication d'un polypeptide pur capable de reconnaître et de se lier sélectivement à l'hormone folliculo-stimulante (FSH) humaine et qui ne produise pas de réponse immunologique chez l'homme, ce polypeptide consistant en :
(a) le récepteur de la FSH humaine ayant la séquence d'acides aminés 1-678 présentée dans SEQ ID N° 2 ;
(b) une mutéine qui conserve au moins 95 % d'identité avec ladite séquence de (a) ;
(c) un polypeptide qui inclut la partie extracellulaire amino-terminale du récepteur de la FSH humaine, incluant la séquence d'acides aminés 1-349 présentée dans SEQ ID N°2 ;
(d) un polypeptide qui inclut une mutéine ladite mutéine conservant au moins 95 % d'identité avec la séquence d'acides aminés 1-349 présentée dans SEQ ID N° 2 ;
(e) un polypeptide qui inclut un fragment de la partie extracellulaire du récepteur de la FSH humaine de (a), ledit fragment conservant les caractéristiques de liaison à la FSH de la partie extracellulaire complète ; ou
(f) un polypeptide qui inclut une mutéine dudit fragment de (e), ladite mutéine conservant au moins 95 % d'identité avec ledit fragment et conservant les mêmes caractéristiques de liaison à la FSH que la partie extracellulaire complète du récepteur de la FSH humaine, ladite partie extracellulaire complète incluant la séquence d'acides aminés 1-349 présentée dans SEQ ID N° 2, comprenant les étapes de mettre en culture des cellules transfectées avec un vecteur d'expression recombinant, le vecteur comprenant de l'ADN codant pour ledit polypeptide, dans un milieu nutritif et à y récupérer le polypeptide.

2. Méthode selon la revendication 1 dans laquelle le polypeptide capable de lier la FSH humaine consiste en la séquence d'acides aminés 1-678 présentée dans SEQ ID N° 2 ou une mutéine qui conserve au moins 95 % d'identité avec ladite séquence.

3. Méthode selon la revendication 1 dans laquelle le polypeptide capable de lier la FSH humaine consiste en la séquence d'acides aminés 1-613 présentée dans SEQ ID N° 2 ou une mutéine qui conserve au moins 95 % d'identité avec ladite séquence.

4. Méthode selon la revendication 1 dans laquelle le polypeptide capable de lier la FSH humaine, conformément à la revendication 1, consiste en la séquence d'acides aminés 1-349 présentée dans SEQ ID N° 2 ou une mutéine qui conserve au moins 95 % d'identité avec ladite séquence.

5. Méthode selon la revendication 1 dans laquelle le polypeptide est une forme mutéine du polypeptide selon la revendication 1 (a), (c) ou (e), dans lequel la partie correspondant à tout ou partie du récepteur de la FSH humaine inclut des substitutions conservatives dans un à dix résidus d'acides aminés, l'emplacement et la nature desdites substitutions étant sélectionnés pour conserver les mêmes caractéristiques de liaison de la FSH du récepteur ou du fragment de récepteur qui n'est pas modifié.

6. Méthode de préparation d'une cellule de mammifère abritant le vecteur d'expression comprenant l'ADN codant pour un polypeptide comme défini dans les revendications 1 à 5, comprenant les étapes de transfecter la cellule avec ledit vecteur d'expression et de mettre en culture les cellules transfectées.

7. Méthode de préparation d'une composition pharmaceutique comprenant un polypeptide comme défini dans l'une quelconque des revendications 1 à 5, comprenant l'étape consistant à formuler le polypeptide avec un porteur pharmaceutiquement acceptable.

8. Utilisation d'un polypeptide comme défini dans l'une quelconque des revendications 1 à 5 pour la préparation d'une composition pharmaceutique pour réduire la bioactivité de la FSH endogène chez un patient.

9. Utilisation d'un polypeptide comme défini dans l'une quelconque des revendications 1 à 5 pour la préparation d'une composition pharmaceutique pour empêcher la croissance et la maturation des follicules chez une patiente.

10. Utilisation d'un polypeptide comme défini dans l'une quelconque des revendications 1 à 5 pour la préparation d'une composition pharmaceutique pour empêcher la spermatogenèse chez un patient.

11. Méthode de dosage de la FSH humaine *ex vivo* utilisant un récepteur de la FSH, **caractérisée en ce qu'**un polypeptide comme défini dans l'une quelconque des revendications 1 à 5 est utilisé comme récepteur de la FSH.
